# FASCICULE DE BREVET EUROPEEN

(11) **EP 3 515 551 B1**
(45) Date de publication et mention de la délivrance du brevet: **21.10.2020**
(21) Numéro de dépôt: 17765462.1
(22) Date de dépôt: 15.09.2017
(51) Int. Cl.: A61B 5/00, A61B 5/107, A61B 5/11, A61F 5/01, A61H 3/00, A61N 1/04, A61N 1/36

(54) **DISPOSITIF DE STIMULATION POUR L'ACTIVATION D'AU MOINS UN MUSCLE INTERVENANT DANS LE RELEVÉ DU PIED**
STIMULATIONSGERÄT ZUR AKTIVIERUNG MINDESTENS EINES ZUR HEBUNG DES FUSSES BETEILIGTEN MUSKELS
STIMULATION DEVICE FOR ACTIVATING AT LEAST ONE MUSCLE INVOLVED IN LIFTING THE FOOT

(30) Priorité: 22.09.2016 FR 1658930
(43) Date de publication de la demande: 31.07.2019
(73) Titulaire: Université Paris-Est Créteil Val de Marne, 94000 Créteil (FR)
(72) Inventeur: MOHAMMED, Samer, 94600 Choisy-le-Roi (FR); AMIRAT, Yacine, 94320 Thiais (FR); GRACIES, Jean-Michel, 75014 Paris (FR)
(74) Mandataire: Regimbeau
(86) Numéro de dépôt international: PCT/EP2017/073235
(87) Numéro de publication internationale: WO 2018/054764

(56) Documents cités:
- US-A- 4 711 242
- US-A1- 2008 234 782
- US-A1- 2012 059 432
- STEPHEN J. PIAZZA AND SCOTT L. DELP: "The Influence of Muscles on Knee Flexion During the Swing Phase of Gait", JOURNAL OF BIOMECHANICS, vol. 29, no. 6, 1996, pages 723-733, XP002770461,

## Description

### Domaine de l'invention

La présente invention concerne un dispositif permettant de stimuler au moins un muscle intervenant dans le relevé du pied d'un utilisateur afin d'activer ledit au moins un muscle et ainsi faciliter la marche dudit utilisateur.

Plus particulièrement, l'invention concerne un dispositif dont la stimulation du ou des muscles intervenant dans le relevé du pied est fonction de l'angle de flexion du genou du membre inférieur correspondant.

L'invention concerne également une unité de traitement pour un tel dispositif de stimulation.

### État de l'art

Un individu atteint d'hémiparésie spastique, par exemple à la suite d'un accident cardio-vasculaire, peut rencontrer des problèmes de locomotion dus à une démarche trop rigide.

En effet, le mouvement de dorsiflexion de la cheville pour le relevé du pied du membre inférieur paralysé de l'individu hémiparétique peut s'avérer trop limité, voir absent.

Un mouvement de dorsiflexion trop limité, voir absent, lorsque l'individu marche augmente le risque pour le pied de buter dans le sol et de causer ainsi une chute.

Une telle insuffisance dans le mouvement de dorsiflexion est due à une mauvaise commande des muscles intervenant dans le relevé du pied. Ces muscles sont les suivants :
- le tibial antérieur (également appelé le jambier antérieur) ;
- l'extenseur d'orteils ;
- les péroniers latéraux, dont le court péronier latéral.

Afin d'aider l'individu à relever le pied du membre inférieur paralysé, il est connu d'utiliser une orthèse de cheville et de pied (AFO) qui maintient mécaniquement ledit pied dudit individu en position relevée.

Toutefois, une telle solution tend à diminuer l'activation volontaire des muscles responsables de la dorsiflexion, réduisant ainsi l'excitabilité corticospinale de la commande d'activation de ces muscles, entrainant ainsi une dépendance de l'individu à l'orthèse.

On connait également des dispositifs permettant de stimuler électriquement les muscles de l'individu responsables de la dorsiflexion, de sorte à activer lesdits muscles lorsqu'il est nécessaire que l'individu relève le pied.

Ainsi, on connait par exemple le document US4796631 qui décrit un dispositif de stimulation électrique des muscles intervenant dans le relevé du pied lors de la marche d'un individu. Le dispositif décrit dans ce document comprend des électrodes qui sont commandées pour stimuler électriquement les muscles lors de la phase d'appui, lorsqu'à la fois un capteur placé sous le talon du pied de l'individu détecte que le talon est au sol, et que l'angle de flexion du genou du même membre inférieur que le pied atteint une valeur minimum préétablie.

Toutefois, un tel dispositif, en plus d'être encombrant, impose que l'individu porte des chaussures afin que le capteur talon puisse être installé sous le talon, et ne permet pas de stimuler de façon satisfaisante les muscles responsables de la dorsiflexion. Le document US 2012/059432 A1 décrit un dispositif de stimulation pour l'activation d'un muscle de relevé du pied pour la marche d'un individu.

On connaît aussi le document *"*Modified implanted drop foot stimulator system with graphical user interface for customised stimulation pulse-width profiles" de T. O'Halloran, M. Haugland, G. M. Lyons, et T. Sinkjaer, qui décrit un dispositif pour la stimulation électrique des muscles intervenant dans le relevé du pied lors de la marche d'un individu. Le dispositif décrit dans ce document comprend une unité de traitement qui commande des électrodes afin que lesdites électrodes stimulent électriquement les muscles de l'individu. L'unité de traitement est configurée pour que les électrodes stimulent les muscles sur 95% du cycle de marche de l'individu à la fois durant la phase d'appui et la phase oscillante, les électrodes commençant la stimulation électrique lorsque le talon du pied de l'individu quitte le sol. De plus, l'unité de traitement est configurée pour que l'intensité de la stimulation augmente lorsque le talon du pied touche le sol à la fin de la phase d'oscillation. Afin de donner l'information à l'unité de traitement du moment où le talon est au contact du sol pour que ladite unité de traitement puisse commander les électrodes en fonction de cette information, le dispositif comprend un capteur de pression disposé sous le talon du pied de l'individu qui détecte lorsque le talon est en appui sur le sol.

Toutefois, là encore, un tel dispositif est encombrant à cause du capteur qui doit être disposé sous le talon de l'individu, impose à l'individu de porter des chaussures afin de placer le capteur sous le talon. La stimulation quasi-permanente est un facteur de fatigabilité musculaire et ne permet pas de stimuler de façon satisfaisante les muscles responsables de la dorsiflexion.

Enfin, on connait également un dispositif de stimulation électrique des muscles intervenant dans le relevé du pied du membre inférieur paralysé lors de la marche de l'individu qui comprend des capteurs mesurant l'inclinaison du segment jambier dudit membre inférieur afin de commander le déclanchement de la stimulation électrique en fonction de l'inclinaison dudit segment jambier.

Toutefois, un tel dispositif ne permet pas de stimuler de façon satisfaisante les muscles responsables de la dorsiflexion.

### Présentation générale de l'invention

Un but de l'invention est de proposer une technique de stimulation pour l'activation d'au moins un muscle intervenant dans le relevé du pied lors de la marche d'un individu qui permet de résoudre les problèmes de l'état de la technique.

Un premier but de l'invention est de déclencher de manière précise la phase de stimulation du ou des muscles, afin d'assurer que le pied soit relevé au moment opportun.

Un deuxième but de l'invention est d'assurer que le relevé du pied est suffisant à la fin de la phase d'oscillation.

Un troisième but de l'invention est de réduire la consommation électrique de la stimulation tout en assurant un relevé du pied suffisant.

Un quatrième but de l'invention est de réduire l'encombrement du dispositif de stimulation, notamment en se passant de l'utilisation d'un capteur de pression disposé sous le talon.

À cet effet, selon un premier aspect de l'invention, il est prévu un dispositif de stimulation pour l'activation d'au moins un muscle intervenant dans le relevé du pied lors de la marche d'un individu comprenant :
- des capteurs configurés pour être mis en place sur un membre inférieur de l'individu et dont des signaux de mesure permettent de calculer un angle de flexion du genou correspondant audit membre inférieur ;
- des électrodes configurées pour être mises en place sur le ou les muscles à activer et propres à stimuler électriquement ledit ou lesdits muscles ;
- une unité de traitement apte à recevoir le signal de mesure des capteurs, ladite unité de traitement présentant des moyens de calcul pour calculer la valeur de l'angle de flexion du genou à partir du signal de mesure reçu des capteurs, et qui comprend des moyens de commande des électrodes reliés aux moyens de calcul et aux électrodes ; où :
   - les moyens de calcul sont configurés pour déterminer la phase d'oscillation dans un cycle de marche de l'individu ;
   - les moyens de commande sont configurés pour :
- activer les électrodes uniquement durant la phase d'oscillation du cycle de marche de l'individu de sorte que les électrodes génèrent une stimulation électrique ayant une intensité fonction de l'angle de flexion du genou,
- activer les électrodes de sorte que les électrodes génèrent une stimulation électrique avec une intensité qui augmente avec la diminution de l'angle de flexion du genou, lors de la ré-extension du genou pendant la phase d'oscillation.

Le dispositif selon l'invention est avantageusement complété par les caractéristiques suivantes, prises seules ou en une quelconque de leurs combinaisons techniquement possibles :
- l'unité de traitement est configurée pour déterminer dans la phase d'oscillation une sous-phase de début d'oscillation et une sous-phase de fin d'oscillation, et activer les électrodes de telle sorte que les électrodes génèrent une stimulation électrique avec une intensité qui augmente entre une première valeur seuil et une deuxième valeur seuil de l'angle du flexion du genou, la première valeur seuil étant atteinte dans la sous-phase de début d'oscillation, et la deuxième valeur seuil étant atteinte dans la sous-phase de fin d'oscillation ;
- l'unité de traitement est configurée pour activer les électrodes de telle sorte que les électrodes génèrent une stimulation électrique avec une intensité qui augmente entre une première valeur seuil et une deuxième valeur seuil, la première valeur seuil étant l'angle de flexion du genou de l'individu au début de la ré-extension du genou, et la deuxième valeur seuil étant l'angle de flexion du genou de l'individu à la fin de la ré-extension du genou survenue dans une sous-phase de fin d'oscillation de la phase d'oscillation ;
- l'unité de traitement est configurée pour activer les électrodes de telle sorte que les électrodes génèrent une stimulation électrique ayant une impulsion d'intensité entre une sous-phase de début d'oscillation et le début de l'extension du genou ;
- l'unité de traitement est configurée pour commander les électrodes pour que lesdites électrodes génèrent des impulsions électriques durant la phase d'oscillation, les impulsions électriques étant définies par une amplitude correspondant à l'intensité du courant appliqué aux électrodes, l'augmentation de l'intensité de la stimulation durant la phase de stimulation par lesdites électrodes étant réalisée en augmentant l'amplitude ;
- l'unité de traitement est configurée pour commander les électrodes de façon à ce que l'intensité de la stimulation augmente de façon linéaire avec la diminution de l'angle de flexion lors de la ré-extension du genou ;
- l'unité de traitement est configurée pour commander les électrodes de façon à ce que l'intensité de la stimulation augmente de façon linéaire avec l'augmentation de l'angle de flexion lors de l'augmentation de l'angle de flexion du genou pendant la phase d'oscillation ;
- l'unité de traitement détermine l'angle de flexion du genou sans être reliée à un capteur sensible à la pression plantaire ;
- les capteurs comprennent une première unité de mesures inertielles et une deuxième unité de mesures inertielles ;
- le dispositif comprend en outre un premier élément d'attache pour attacher la première unité de mesures inertielles à une cuisse du membre inférieur, et un deuxième élément d'attache pour attacher la deuxième unité de mesures inertielles à un segment jambier dudit membre inférieur.

Selon un deuxième aspect, l'invention concerne également une unité de traitement pour l'activation d'au moins un muscle intervenant dans le relevé du pied lors de la marche d'un individu, configurée pour recevoir des signaux de mesure de capteurs et présentant :
- des moyens de calcul pour déterminer la valeur de l'angle de flexion du genou du membre inférieur de l'individu à partir dudit au moins un signal de mesure reçu, et pour déterminer la phase d'oscillation dans un cycle de marche de l'individu ;
- des moyens de commande reliés aux moyens de calcul pour commander des électrodes en fonction de la valeur de l'angle de flexion du genou déterminée ;
en ce que les moyens de commande génèrent un signal de commande déclenchant une phase de stimulation par lesdites électrodes uniquement durant la phase d'oscillation du cycle de marche de l'individu :
- de sorte que les électrodes génèrent une stimulation électrique dont l'intensité est fonction de l'angle de flexion du genou et qui augmente avec la diminution de l'angle de flexion du genou, lors d'une ré-extension du genou pendant la phase d'oscillation.

Un procédé de traitement est décrit pour une unité de traitement selon le deuxième aspect, comprenant les étapes suivantes :
- recevoir un signal de mesures de capteurs permettant la mesure d'un angle de flexion du genou d'un membre inférieur d'un individu ;
- déterminer la valeur de l'angle de flexion du genou à partir du signal de mesure reçu à l'étape précédente et déterminer la phase d'oscillation dans un cycle de marche de l'individu ;
- générer un signal de commande uniquement durant la phase d'oscillation détectée lors de l'étape précédente, de sorte que :
   - les électrodes génèrent une stimulation électrique qui augmente avec la diminution de l'angle de flexion du genou, lors de la ré-extension du genou pendant la phase d'oscillation.

Selon un troisième aspect, l'invention concerne également un produit programme d'ordinateur comprenant des instructions de code de programme pour l'exécution des étapes du procédé de traitement d'un signal suivant le troisième aspect.

Selon un quatrième aspect, l'invention concerne également un support utilisable dans un ordinateur sur lequel est enregistré le produit programme d'ordinateur selon le quatrième aspect.

Un procédé de stimulation est décrit pour l'activation d'au moins un muscle intervenant dans le relevé du pied lors de la marche d'un individu comprenant les étapes suivantes :
- mesurer un angle de flexion d'un genou de l'individu et détecter la phase d'oscillation du cycle de marche de l'individu à partir de la valeur de l'angle de flexion du genou mesurée ;
- déclencher une phase de stimulation électrique du ou des muscles uniquement durant la phase d'oscillation d'un cycle de marche de l'individu, la stimulation électrique étant fonction de l'angle de flexion du genou, et augmentant avec la diminution de l'angle de flexion du genou lors de la ré-extension du genou pendant la phase d'oscillation.

Selon une caractéristique supplémentaire du procédé de stimulation, la stimulation est déclenchée de façon périodique pour n cycles de marche de l'individu parmi N cycles de marche considérés, n entier naturel supérieur ou égal à 2 et inférieur strictement à N, N nombre de cycle de marche.

Par exemple, les moyens de commande peuvent envoyer une stimulation un cycle de marche sur deux, pour que l'utilisateur puisse essayer de lui-même de lever le pied, avant d'être à nouveau aider par la stimulation commandée par les moyens de commande aux électrodes, et ainsi de suite.

Une utilisation du dispositif selon le premier aspect de l'invention est décrite pour des patients neurologiques atteints de parésie spastique.

### Présentation des figures

D'autres caractéristiques, buts et avantages de l'invention apparaitront à la lecture de la description ci-après de différents modes de réalisation représentés dans les dessins suivants :
- la figure 1a représente de manière schématique un dispositif de stimulation disposé sur un individu ;
- la figure 1b représente de manière schématique un dispositif de stimulation disposé sur un membre inférieur d'un individu ;
- la figure 2 représente sous forme de courbes l'évolution de l'angle de flexion du genou et l'angle de flexion de la cheville lors du cycle de marche d'un individu ;
- la figure 3a représente l'évolution de l'angle de flexion du genou, l'évolution de l'intensité du courant de stimulation créé par des électrodes, et la réponse d'un capteur de pression situé sous le talon pour un individu sain ;
- la figure 3b représente l'évolution de l'angle de flexion du genou, l'évolution de l'intensité du courant de stimulation créé par des électrodes, et la réponse d'un capteur de pression situé sous le talon pour un individu atteint d'hémiparésie spastique ;
- la figure 4 représente de manière schématique des impulsions électriques créées par des électrodes pour stimuler un ou des muscles ;
- la figure 5a représente une variante possible de fonction entre l'angle de flexion du genou et l'intensité du courant de stimulation ;
- la figure 5b représente une variante supplémentaire de fonction possible entre l'angle de flexion du genou et l'intensité du courant de stimulation ;
- la figure 5c représente encore une autre variante de fonction possible entre l'angle de flexion du genou et l'intensité du courant de stimulation ;
- la figure 6 représente sur un cycle de marche complet la comparaison de la variation de l'angle de flexion de la cheville entre des individus qui ne sont pas stimulés, des individus stimulés dont l'intensité de stimulation est constante durant la phase de stimulation, et des individus stimulés ;
- la figure 7 représente une vue plus précise de la figure 6 entre 0% et 10% du cycle de marche ;
- la figure 8 représente une vue plus précise de la figure 6 entre 50% et 100% du cycle de marche ;
- la figure 9 représente une mise en œuvre possible d'un procédé de traitement d'un signal pour une unité de traitement ;
- la figure 10 représente une mise en oeuvre possible d'un procédé de stimulation pour l'activation d'au moins un muscle intervenant dans le relevé du pied lors de la marche d'un individu.

### Description de l'invention

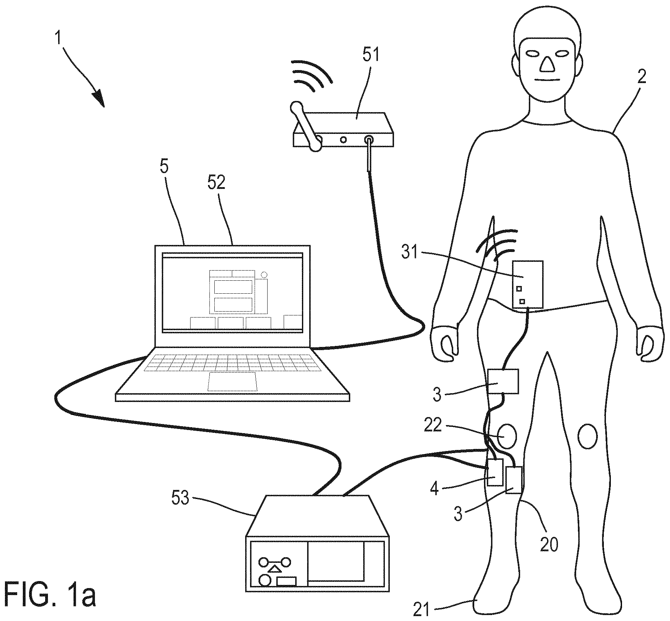

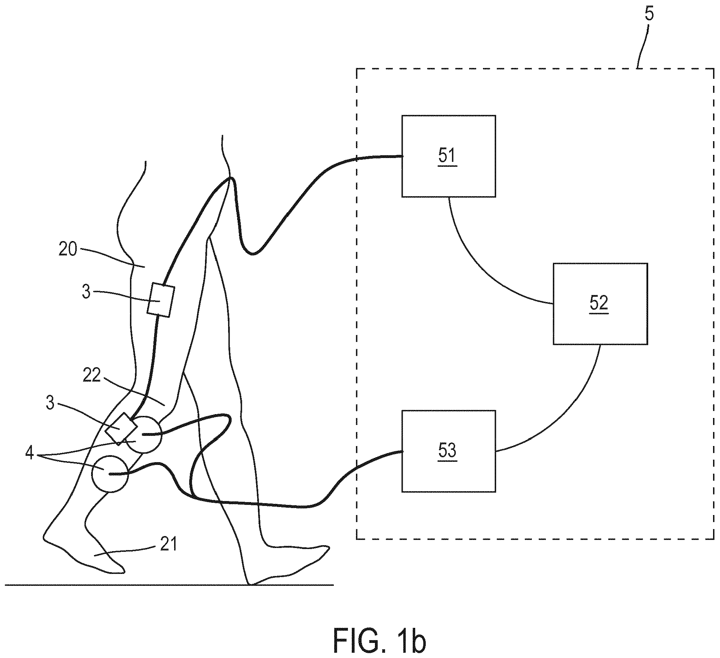

Comme illustré sur la figure 1a et la figure 1b, le dispositif 1 de stimulation pour l'activation d'au moins un muscle intervenant dans le relevé du pied 21 d'un membre inférieure 20 d'un individu 2 lors de la marche dudit individu 2 comprend les éléments suivant :
- des capteurs 3 disposés sur le membre inférieur 20 déficient de l'individu 2 qui mesurent l'angle de flexion du genou 22 dudit membre inférieur 20 ;
- au moins deux électrodes 4 disposées sur le membre inférieur 20 en regard du ou des muscles à activer afin de stimuler électriquement ledit ou lesdits muscles ;
- une unité de traitement 5 qui contrôle les électrodes 4 en fonction de la valeur de l'angle de flexion du genou 22 mesurée par les capteurs 3.

Selon une variante possible, les capteurs 3 peuvent être des unités de mesures inertielles (également appelées IMU, pour « Inertial Measurement Unit » selon la terminologie anglo-saxonne). Dans cette variante, une première unité de mesures inertielles est placée sur la cuisse de l'individu 2, et une deuxième unité de mesures inertielles est placée sur le segment jambier de l'individu 2. Les unités de mesures inertielles comprennent selon une variante possible, trois gyromètres et trois accéléromètres. Les trois gyromètres de chaque unité de mesures inertielles permettent de mesurer les trois composantes de la vitesse angulaire de la cuisse du segment jambier, et les trois accéléromètres de chaque unité de mesures inertielles permettent de mesurer les trois composantes de l'accélération linéaire de ladite cuisse et dudit segment jambier.

L'angle de flexion du genou 22 mesuré par les capteurs 3 est l'angle formé par l'inclinaison du segment jambier par rapport à la cuisse. Ainsi, lorsque le membre inférieur 20 de l'individu 2 est tendu, c'est-à-dire que le segment jambier et la cuisse sont alignés, alors l'angle de flexion du genou 22 est égale à 0°.

Les capteurs 3 émettent, via un émetteur 31, un signal de mesure qui est transmis à l'unité de traitement 5. L'unité de traitement 5 comprend un récepteur 51 qui capte le signal de mesure émis par l'émetteur 31 des capteurs 3, l'unité de traitement 5 étant reliée aux capteurs 3, grâce audit récepteur 51. Le signal de mesure peut être transmis des capteurs 3 vers l'unité de traitement 5 de manière filaire ou de manière sans fil, par exemple par wifi.

L'unité de traitement 5 comprend des moyens de calcul 52 comprenant un processeur ainsi qu'une mémoire. Les moyens de calcul 52 traitent le signal de mesure envoyé par les capteurs 3 et calculent la valeur de l'angle de flexion du genou 22 à partir dudit signal de mesure. Les capteurs 3 et l'unité de traitement 5 permettent de mesurer en temps réel l'angle de flexion du genou 22.

De plus, les moyens de calcul 52 détectent les différentes phases du cycle de marche de l'individu à partir de la variation de la valeur de l'angle de flexion du genou 22 au cours du temps ou à partir d'autres paramètres connus de l'état de l'art.

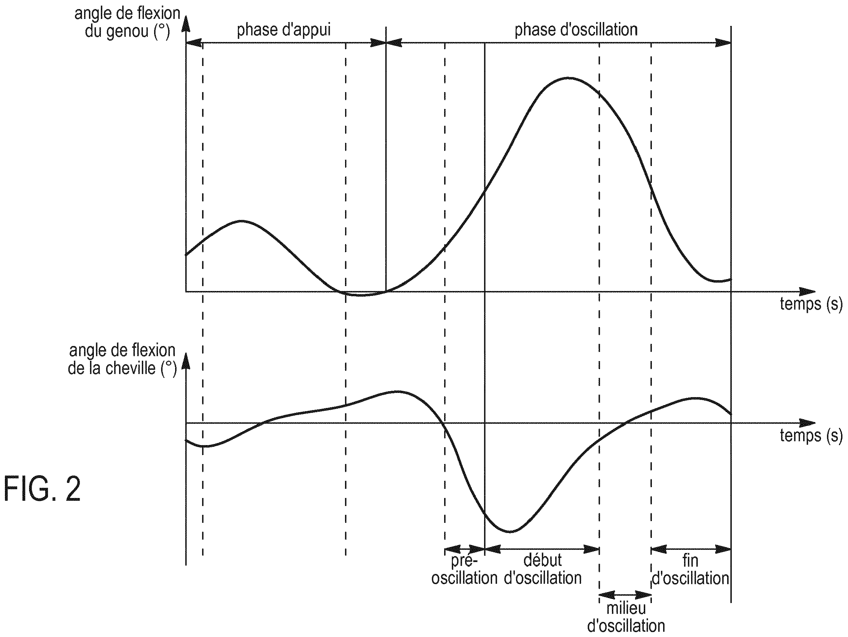

En effet, comme visible sur la figure 2, le cycle de marche d'un individu est divisé en deux grandes phases : la phase d'appui (ou « stance phase » selon la terminologie anglo-saxonne) pendant laquelle le pied 21 reste au sol et l'individu soulève et avance l'autre pied, et la phase d'oscillation (ou « swing phase » selon la terminologie anglo-saxonne) pendant laquelle l'individu soulève le pied 21 pour l'amener devant lui et pose ledit pied 21 au sol. La phase d'appuie se termine et la phase d'oscillation commence lorsque le talon du pied 21 quitte le sol.

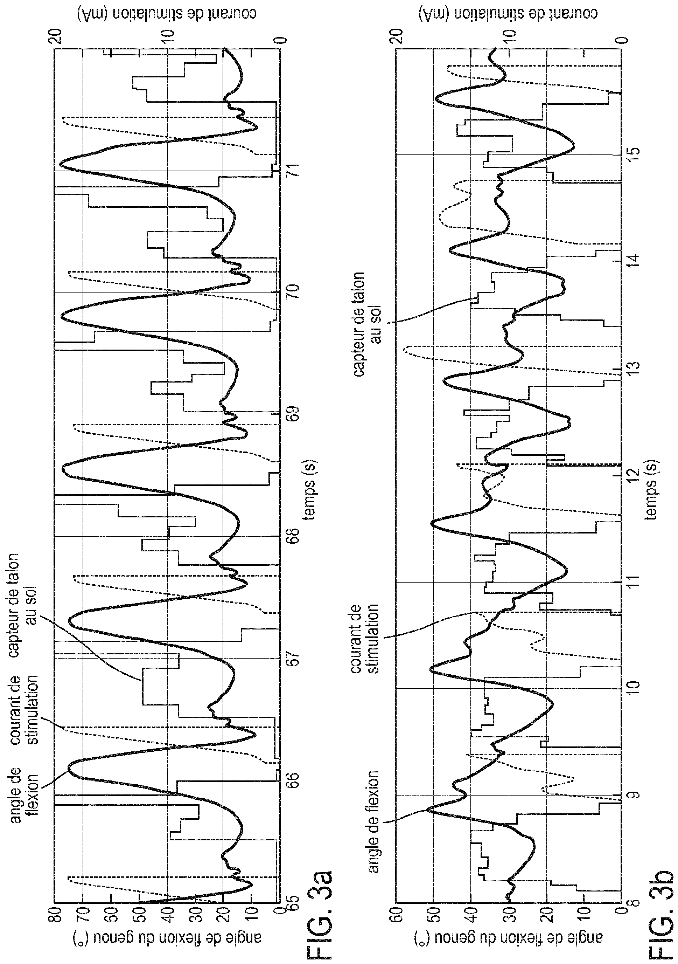

Pendant la phase d'appui, comme visible sur la figure 2, l'individu a un mouvement de flexion du genou 22 de faible amplitude qui est suivi d'un mouvement d'extension du genou 22 de faible amplitude également. Pendant la phase d'oscillation, l'individu 2 a un mouvement de flexion du genou 22 de forte amplitude qui est suivi d'un mouvement d'extension du genou 22 de forte amplitude. Le mouvement d'extension du genou 22 de forte amplitude durant la phase d'oscillation est appelé « ré-extension du genou 22 » suivant la terminologie du domaine technique. Cette ré-extension du genou 22 correspond au mouvement que fait l'individu 2 lorsqu'il a le pied 21 en l'air et qu'il étend le membre inférieur 20 jusqu'à poser le talon du pied 21 au sol. La ré-extension commence par exemple à partir de 50° sur la figure 3b à partir duquel l'angle de flexion diminue, à la phase de « mid-swing » selon la terminologie anglo-saxonne.

Comme illustré sur la figure 2, les moyens de calcul 52 de l'unité de traitement 50 détectent la phase d'appui et la phase d'oscillation en détectant, pour chaque cycle, deux pics de différentes valeurs dans la variation de la valeur de l'angle de flexion du genou 22 au cours du temps, un premier pic de faible valeur correspondant à la phase d'appui, et un second pic de valeur élevée correspondant à la phase d'oscillation.

Par ailleurs, les moyens de calcul 52 de l'unité de traitement 5 détectent les différentes sous-phases qui composent les phases d'appui et d'oscillation du cycle de marche de l'individu 2. Ainsi, les moyens de calcul 52 détectent :
- une sous-phase de pré-oscillation (ou « pre-swing » selon la terminologie anglo-saxonne) qui débute la phase d'oscillation en faisant la transition avec la fin de la phase d'appui. La sous-phase de pré-oscillation commence lorsque le talon du pied 21 quitte le sol, et se termine lorsque les orteils du pied 21 quittent le sol ;
- une sous-phase de début d'oscillation (ou « initial-swing » selon la terminologie anglo-saxonne) qui suit la sous-phase de pré-oscillation et dans laquelle la valeur de l'angle de flexion atteint son maximum correspondant au pic de valeur élevée détectée précédemment. La sous-phase de début d'oscillation commence lorsque les orteils du pied 21 quittent le sol, et se termine lorsque le pied 21 de l'individu 2 est au même niveau que l'autre pied dudit individu 2 ;
- une sous-phase de milieu d'oscillation (ou « mid-swing » selon la terminologie anglo-saxonne) qui suit la sous-phase de début d'oscillation et qui correspond au début de la ré-extension du genou 22. La sous-phase de milieu d'oscillation commence lorsque les deux pieds de l'individu 2 sont au même niveau, et se termine lorsque le segment jambier du membre inférieur 20 est vertical (perpendiculaire par rapport au sol) ;
- une sous-phase de fin d'oscillation (ou « terminal-swing » selon la terminologie anglo-saxonne) qui suit la sous-phase de milieu d'oscillation et qui correspond à la fin de la ré-extension du genou 22, ladite sous-phase de fin d'oscillation se terminant par la pose du talon au sol du pied 21 de l'individu 2. À la suite de la sous-phase de fin d'oscillation commence une nouvelle phase d'appui d'un nouveau cycle de marche.

L'unité de traitement 5 comprend des moyens de commande 53 des électrodes 4.Les moyens de commande 53 génèrent un signal de commande qui active les électrodes 4 pour que lesdites électrodes 4 déclenchent une phase de stimulation du ou des muscles uniquement pendant la phase d'oscillation du cycle de marche du membre inférieur 20 déficient de l'individu 2.

Afin que les moyens de commande 53 activent les électrodes 4 uniquement pendant la phase d'oscillation des cycles de marche de l'individu 2, lesdits moyens de commande 23 génèrent le signal de commande uniquement lorsque la valeur de l'angle de flexion du genou 22 atteint une première valeur seuil. Cette première valeur seuil peut être adaptée pour qu'elle corresponde à une position du genou 22 lorsque l'individu est dans la sous-phase de début d'oscillation.

La valeur du premier seuil est réglée par le kinésithérapeute au moment de l'installation du dispositif 1 sur l'individu 2. Comme visible sur les figures 2, 3a et 3b, la valeur du premier seuil doit être d'une part suffisamment élevée pour ne pas que la phase de stimulation se déclenche lors de la phase d'appui, et d'autre part suffisamment faible pour que la phase de stimulation se déclenche lors de la phase d'oscillation. Dit autrement, il est nécessaire que le premier seuil soit d'une part supérieur à la valeur du premier pic (le pic de la phase d'appui) que fait la valeur de l'angle de flexion du genou 22, et d'autre part inférieur ou égal au deuxième pic (le pic de la phase d'oscillation) que fait la valeur de l'angle de flexion du genou 22.

La valeur du premier seuil peut être égale à la valeur de l'angle de flexion du genou 22 au début de la ré-extension du genou 22 durant la phase d'oscillation, c'est-à-dire à la valeur du second pic correspondant à la phase d'oscillation.

De plus, afin que les moyens de commande 53 activent les électrodes 4 uniquement pendant la phase d'oscillation, lesdits moyens de commande 23 arrêtent de générer le signal de commande activant les électrodes 4 lorsque la valeur de l'angle de flexion du genou 22 est inférieure à une deuxième valeur seuil qui est inférieure à la première valeur seuil. Cette deuxième valeur seuil peut être choisie afin qu'elle corresponde à une position du genou 22 lorsque l'individu est dans la sous-phase de fin d'oscillation.

La valeur du deuxième seuil peut être égale à la valeur de l'angle de flexion du genou 22 à la fin de la ré-extension du genou 22.

Comme visible sur la figure 3b, l'individu 2 étant hémiparétique, le genou 22 ne fléchit pas de manière normale lors du cycle de marche dudit individu 2. Ainsi, l'individu 2 peut avoir un membre inférieur 20 très raide et ne fléchir que très légèrement le genou 22 lorsqu'il marche, de sorte que l'angle de flexion du genou 22 reste toujours très proche de 0° durant tout le cycle de marche. Selon un autre cas possible, l'individu peut garder en permanence le genou 22 fléchi, de sorte que l'angle de flexion du genou 22 ne sera jamais égal à 0°. C'est pourquoi il est nécessaire que le kinésithérapeute adapte la valeur du premier seuil et du deuxième seuil en fonction de l'individu. De plus, comme visible sur les figures 3a et 3b, la phase d'appui du cycle de marche et la phase d'oscillation de l'individu 2 peuvent être déformée par rapport aux phases du cycle de marche d'un individu sain.

Selon une variante possible, au lieu d'arrêter l'activation des électrodes 4 en dessous de la deuxième valeur seuil, l'activation des électrodes 4 est arrêtée lorsque les moyens de calcul 52 de l'unité de traitement 50 détectent un minimum dans la variation de la valeur de l'angle de flexion du genou 22. Le minium est détecté lorsque la valeur de l'angle de flexion du genou 22 augmente après avoir diminué durant la ré-extension dudit genou 22.

Selon une variante supplémentaire, l'arrêt de la phase de stimulation durant laquelle les électrodes 4 sont activées est réalisé lorsque d'une part la valeur de l'angle de flexion passe le deuxième seuil, et d'autre part lorsque le minium est détecté. Une telle variante permet d'une part de prendre en compte la variabilité de la valeur minimum atteinte par l'angle de flexion du genou 22 au cours de différents cycles de marche qui se suivent, et d'autre part permet de ne pas prendre en compte les minimums dans la variation de l'angle de flexion du genou 22 au cours de la phase d'oscillation qui sont dus à des spasmes du membre inférieur 20 de l'individu 2 et qui se produisent avant la fin de la ré-extension du genou 22.

Selon une variante préférentielle, la phase de stimulation du ou des muscles par les électrodes 4 est réalisée uniquement lors de la ré-extension du genou 22 lors de la phase d'oscillation, l'intensité de la stimulation augmentant progressivement pendant la ré-extension dudit genou 22.

Toutefois, selon une autre variante possible, l'unité de traitement 5 déclenche la phase de stimulation avant la ré-extension du genou 22, par exemple pendant la sous-phase de pré-oscillation durant laquelle le genou 22 fléchi. Dans cette variante, il est possible que la phase de stimulation commence par un pic d'intensité de stimulation, l'intensité de stimulation diminuant progressivement jusqu'à la ré-extension du genou 22 (c'est-à-dire jusqu'à ce que la valeur de l'angle de flexion du genou 22 atteigne le premier seuil) et à partir de ladite ré-extension du genou 22 l'intensité augmente progressivement jusqu'à la fin de la phase d'oscillation.

La phase de stimulation par les électrodes 4 est déclenchée en faisant passer un courant électrique par les électrodes 4 qui force le ou les muscles traversés par ledit courant électrique à se contracter. Les électrodes 4, au moins deux, sont donc disposées en regard du ou des muscles à stimuler. Les dites électrodes 4 sont placées par le kinésithérapeute lors de la mise en place du dispositif 1 sur l'individu 2 afin d'adapter le placement des électrodes 4 en fonction de la morphologie de l'individu 4. Les électrodes 4 sont donc disposées en regard du tibial antérieur, et/ou de l'extenseur d'orteils, et/ou des péroniers latéraux. De plus, pour une position des électrodes 4 donnée, la modification de l'intensité du courant électrique envoyé par les électrodes permet également de modifier les muscles stimulés car une faible intensité ne stimule que le ou les muscles situés à proximité de la peau (et donc des électrodes 4), tandis qu'une forte intensité permet de stimuler un ou des muscles qui sont situés plus en profondeur du membre inférieur (et qui sont donc relativement éloignés des électrodes 4).

L'intensité du courant envoyé par les électrodes 4 est réglée par le kinésithérapeute afin d'adapter l'intensité du courant envoyé par les électrodes 4 en fonction de la morphologie de l'individu, de son niveau de paralysie, et de son endurance. Par exemple, pour une personne très corpulente il est nécessaire que l'intensité du courant envoyé par les électrodes 4 soit plus importante que pour une personne moins corpulente afin de stimuler de la même manière le ou les muscles désirés. L'intensité du courant des impulsions électriques est de préférence comprise entre 20mA et 40mA.

Afin de faire passer un courant électrique par les électrodes 4, le dispositif 1 comprend une batterie reliée auxdites électrodes 4 qui stocke de l'énergie électrique afin d'alimenter électriquement les électrodes 4 uniquement lors de la phase de stimulation.

L'intensité de la stimulation réalisée par les électrodes 4 durant la phase de stimulation est fonction de l'angle de flexion du genou 22. Cela permet d'assurer une forte intensité de stimulation à la fin de la phase d'oscillation, et plus précisément à la fin de la ré-extension du genou 22, par rapport au reste de la phase de stimulation.

Le fait d'augmenter ainsi l'intensité de la stimulation durant la phase de stimulation permet que le pied 21 soit d'avantage relevé à la fin de la phase de stimulation. En effet, la demanderesse s'est aperçu qu'il est préférable que le pied soit d'avantage relevé à la fin de la phase d'oscillation (sous-phase de fin d'oscillation), ce qui correspond à la fin de la ré-extension du genou 22.

En effet, dans certaines stimulations réalisées dans l'art antérieur, l'intensité de la stimulation ne varie pas durant la phase de stimulation, de sorte que la courbe de l'intensité de stimulation au cours du temps forme un créneau dans lequel l'intensité est à 0 en dehors de la phase de stimulation, et l'intensité est à 1 pendant la phase de stimulation.

Ces solutions de l'état de la technique imposent le fait de stimuler fortement les muscles dès le début de la phase de stimulation, ce qui d'une part consomme plus d'énergie, et d'autre part fatigue plus rapidement l'individu 2.

L'invention permet ainsi de limiter la consommation électrique du dispositif 1, permettant ainsi de gagner en autonomie et de réduire l'encombrement de la batterie alimentant les électrodes 4 en électricité. De plus l'invention permet de réduire la fatigue de l'individu 1 en ne stimulant le ou les muscles du membre inférieur 20 que lorsque cela est utile, et avec une intensité adaptée en fonction du mouvement du membre inférieur 20 qu'est en train de réaliser ledit individu 1.

De plus, le fait d'augmenter l'intensité de la stimulation durant la ré-extension du genou 22 permet de contrer le réflexe de contraction des muscles opposés au mouvement de dorsiflexion, assurant ainsi que le pied 21 soit suffisamment relevé lorsque l'individu 2 pose ledit pied 21 au sol.

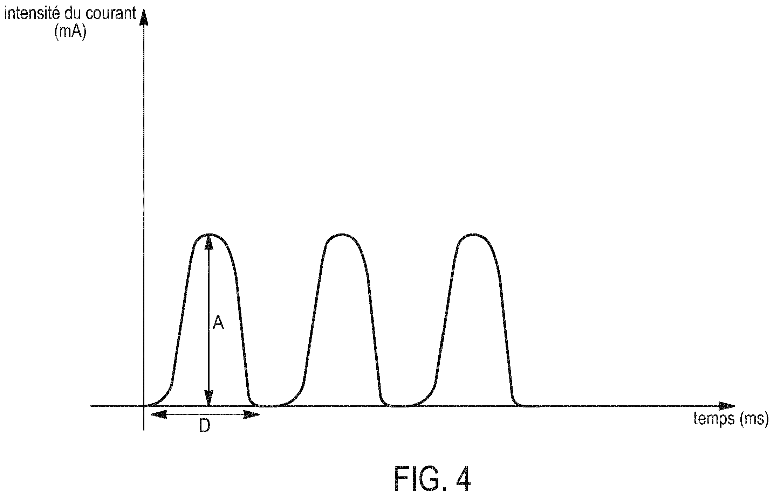

Comme illustrée sur la figure 4, la stimulation électrique des électrodes 4 est de préférence réalisée en appliquant une succession d'impulsions électriques sur le ou les muscles avec les électrodes 4. Les impulsions électriques transmises par les électrodes 4 sont définies par les paramètres suivants :
- Une amplitude A, qui correspond à l'intensité du courant envoyé pendant l'impulsion. A est de préférence compris entre 20mA et 40mA.
- Une fréquence, qui correspond au nombre d'impulsions transmises par unité de temps. La fréquence est de préférence comprise entre 10Hz et 30Hz, et est encore plus de préférence égale à 20Hz. Avec une fréquence de 20Hz, les muscles atteints par les impulsions électriques n'ont pas le temps de se relâcher.
- une durée D, qui correspond au temps durant lequel chaque impulsion est appliquée. D est de préférence comprise entre 2 et 10 µs, et est encore plus de préférence égale à 5 µs.

L'intensité de la stimulation électrique peut être augmentée (ou diminuée) en augmentant (ou diminuant) l'amplitude A des impulsions, et/ou la fréquence desdites impulsions, et/ou la durée D desdites impulsions.

Les courbes d'intensité de stimulation représentées sur les figures 3a et 3b sont formées en reliant les pics des impulsions électriques générées par les électrodes 4.

L'intensité de la stimulation par les électrodes 4 lors de la phase de stimulation est fonction de l'angle de flexion du genou 22, l'intensité de la stimulation augmentant lorsque l'angle de flexion du genou 22 diminue lors de la ré-extension du genou 22.

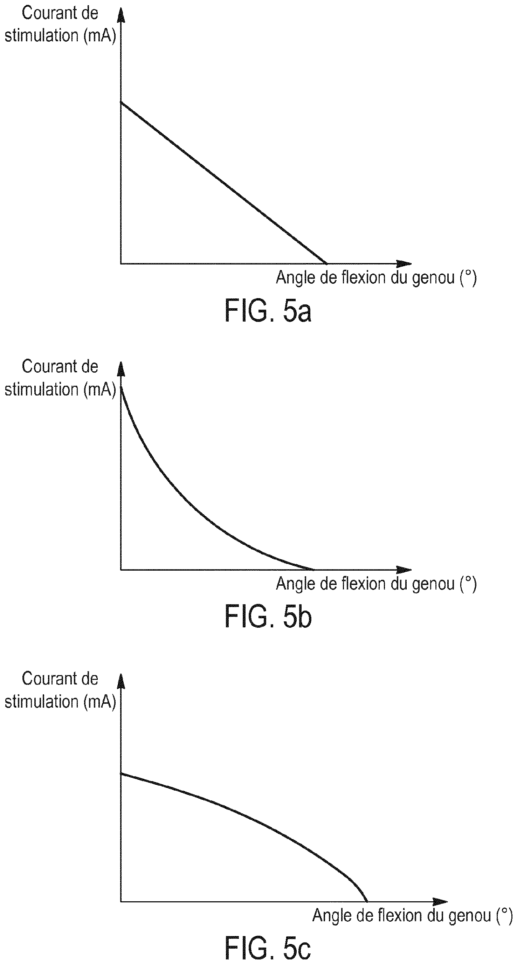

Dans l'exemple donné dans la figure 5a, l'intensité de la stimulation est inversement proportionnelle à l'angle de flexion du genou 22, l'intensité augmentant donc de manière linéaire par rapport à l'angle de flexion.

Dans l'exemple donné dans la figure 5b, l'intensité de la stimulation augmente de manière hyperbolique lorsque l'angle de flexion du genou 22 diminue.

Dans l'exemple donné dans la figure 5c, l'intensité de la stimulation augmente de manière logarithmique lorsque l'angle de flexion du genou 22.

D'autres fonctions sont également possibles, l'intensité de la stimulation pouvant augmenter de manière exponentielle ou parabolique lorsque l'angle de flexion du genou 22 diminue.

Dans les exemples donnés aux figures 5a, 5b et 5c, l'augmentation de l'intensité de stimulation est réalisée en augmentant l'intensité du courant électrique transmis par les électrodes 4 durant la phase de stimulation, par exemple en augmentant l'amplitude A des impulsions électriques transmises par les électrodes). Toutefois, l'augmentation de l'intensité de la stimulation peut également être obtenue en augmentant la fréquence des impulsions électriques, et/ou la durée D desdites impulsions électriques.

Selon une variante possible, l'intensité de stimulation commandée par l'unité de traitement 50 aux électrodes 4 augmente lorsque l'angle de flexion du genou 22 augmente durant la sous-phase de pré-oscillation et la sous-phase de début d'oscillation.

Comme visible sur les figures 3a et 3b qui représentent différentes phases de stimulation en utilisant une variante possible du dispositif 1 selon l'invention sur d'une part un individu sain (figure 3a), et d'autre part un individu hémiparétique (figure 3b), la phase de stimulation est réalisée durant la ré-extension du genou 22. L'intensité de la stimulation (ici l'intensité du courant généré par les électrodes 4) est maximum juste à la fin du mouvement de ré-extension du genou 22 avant que le talon ne touche sol. Le talon touche le sol lorsque la courbe représentant le capteur de talon au sol est supérieure 0. Le capteur de talon au sol est ici uniquement utilisé pour rendre visible sur les figures 3a et 3b le moment où les individus posent leur talon au sol, le dispositif 1 selon l'invention n'a toutefois pas besoin d'un tel capteur de talon au sol pour fonctionner. En effet, le dispositif 1 peut détecter les différentes phases du cycle de marche des individus uniquement à partir des capteurs 3.

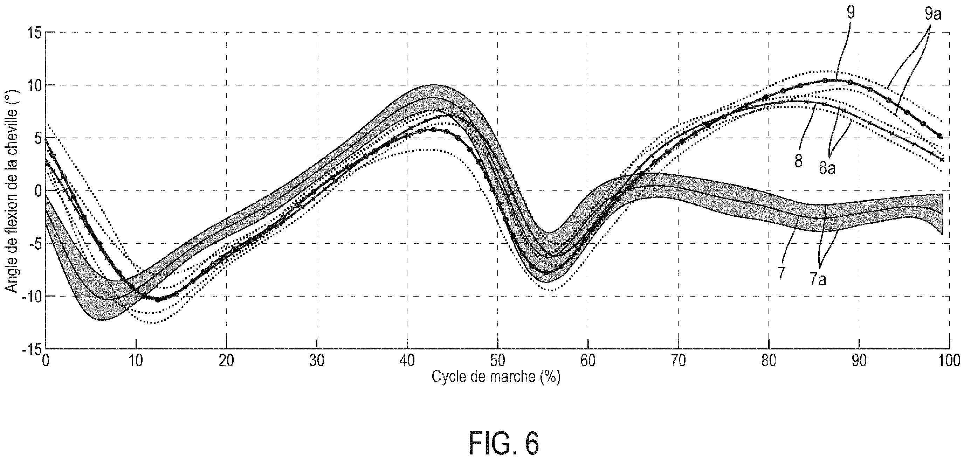

La figure 6 représente l'angle de flexion de la cheville d'individus 2 tout au long d'un cycle de marche, de sorte à comparer l'utilisation du dispositif 1 selon la variante des figures 1a et 1b avec d'une part un dispositif qui génère une phase de stimulation durant laquelle l'intensité de stimulation ne varie pas, et d'autre part avec un individu qui n'est pas stimulé. La variante de dispositif 1 utilisée ici fait varier l'intensité de stimulation en faisant varier l'intensité du courant électrique envoyé par les électrodes 4 durant la phase de stimulation.

La courbe 7 représente la moyenne de l'angle de flexion de la cheville pour des individus non stimulés, et les courbes 7a représentent l'écart type de l'angle de flexion de la cheville pour les individus non stimulés.

La courbe 8 représente la moyenne de l'angle de flexion de la cheville pour des individus qui ont été stimulés avec une intensité de stimulation constante durant la phase de stimulation, et les courbes 8a représentent l'écart type de l'angle de flexion de la cheville pour les individus qui ont été stimulés avec une intensité de stimulation constante durant la phase de stimulation.

La courbe 9 représente la moyenne de l'angle de flexion de la cheville pour des individus qui ont été stimulés avec le dispositif selon l'invention, c'est-à-dire avec une intensité de stimulation qui augmente durant la phase de stimulation et dont le maximum intervient en sous-phase de fin d'oscillation et correspond à l'intensité de stimulation utilisée pour la stimulation à intensité constante de la courbe 8. Les courbes 9a représentent l'écart type l'angle de flexion de la cheville pour des individus qui ont été stimulés avec le dispositif selon l'invention.

Comme visible sur la figure 6, la stimulation électrique permet d'augmenter sensiblement l'amplitude du mouvement de dorsiflexion pendant la phase d'oscillation. De plus, le dispositif selon l'invention permet aux individus 2 de relever d'avantage le pied 21, l'amplitude du mouvement de dorsiflexion étant supérieur à la fin de la phase d'oscillation. Le fait que l'amplitude du mouvement de dorsiflexion avec le dispositif selon l'invention soit supérieure est d'autant plus remarquable que la puissance électrique envoyée par les électrodes est inférieure à la puissance électrique envoyée par les électrodes du dispositif dans lequel l'intensité de stimulation ne varie pas.

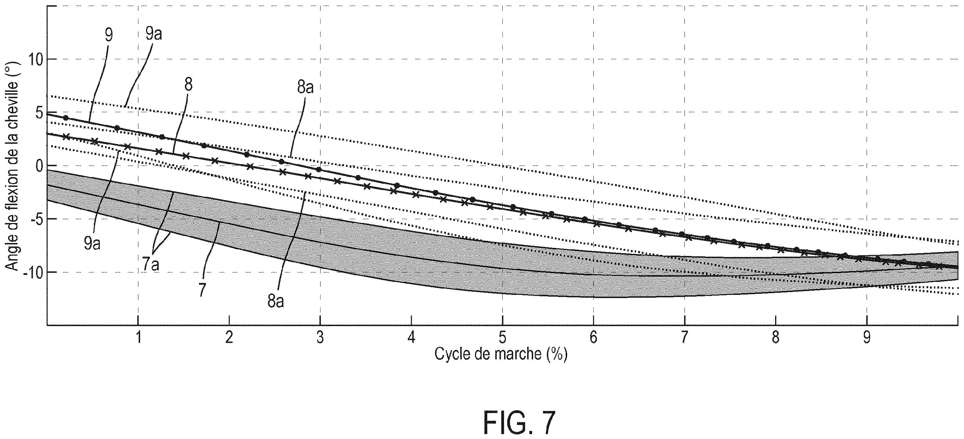

Comme illustré sur la figure 7, qui détaille le début de la phase d'appui, le dispositif 1 selon l'invention permet d'avoir une dorsiflexion plus importante de la cheville au début de la phase d'appui par rapport au dispositif pour lequel l'intensité de stimulation ne varie pas. La moyenne de la différence d'angle de flexion de la cheville sur cette partie du cycle de marche (de 0% à 10% du cycle de marche), entre le cas où l'individu n'est pas stimulé et le cas où l'individu est stimulé avec le dispositif selon l'invention, est de 5,134°. La moyenne de la différence d'angle de flexion de la cheville sur cette partie du cycle de marche, entre le cas où l'individu n'est pas stimulé et le cas où l'individu est stimulé sans faire varier l'intensité de la stimulation, est quant à elle de 4,451°.

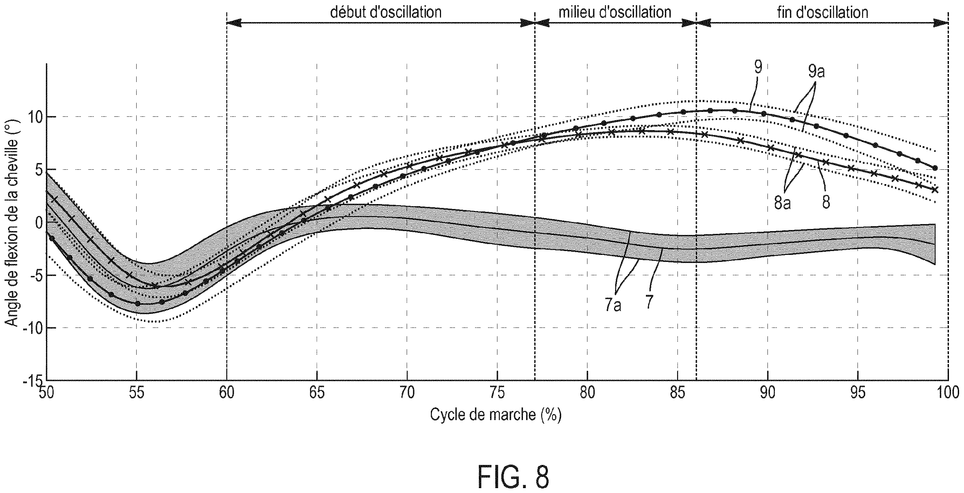

Comme illustré sur la figure 8, qui détaille la phase d'oscillation, le dispositif 1 selon l'invention permet d'avoir un mouvement de dorsiflexion plus important de la cheville uniquement pendant la sous-phase de milieu d'oscillation (de 77% à 86% du cycle) et la sous-phase de fin d'oscillation (de 86% à 100% du cycle). En effet, sur la sous-phase de début d'oscillation (de 60% à 77% du cycle), le mouvement de dorsiflexion est plus important avec le dispositif dans lequel l'intensité de stimulation est constante. Toutefois, cette différence dans l'amplitude du mouvement de dorsiflexion est faible. La moyenne de la différence d'angle de flexion pendant la sous-phase de début d'oscillation (de 60% à 77%) entre le cas où l'individu n'est pas stimulé et le cas où l'individu est stimulé sans faire varier l'intensité de stimulation est de 3,184°. La moyenne de la différence d'angle de flexion pendant la sous-phase de début d'oscillation (de 60% à 77% du cycle) entre le cas où l'individu n'est pas stimulé et le cas où l'individu est stimulé en faisant varier l'intensité de stimulation est de 2,642°.

Pour la sous-phase de milieu d'oscillation (77% à 86% du cycle), la moyenne de la différence d'angle de flexion entre le cas où l'individu n'est pas stimulé et le cas où l'individu est stimulé en faisant varier l'intensité de stimulation est de 10,744°, tandis que la moyenne de la différence d'angle de flexion entre le cas où l'individu n'est pas stimulé et le cas où l'individu est stimulé avec une intensité de stimulation constante est de 9,917°.

Pour la sous-phase de fin d'oscillation (86% à 100% du cycle), la moyenne de la différence d'angle de flexion entre le cas où l'individu n'est pas stimulé et le cas où l'individu est stimulé en faisant varier l'intensité de stimulation est de 10,721°, tandis que la moyenne de la différence d'angle de flexion entre le cas où l'individu n'est pas stimulé et le cas où l'individu est stimulé avec une intensité de stimulation constante est de 8,047°.

Par ailleurs, comme illustré sur le tableau 1 qui compare la durée moyenne mis par un individu équipé d'un dispositif de stimulation des muscles dorsiflexeurs pour parcourir une distance de 10 mètres en marchant, la vitesse moyenne de déplacement de l'individu, et la longueur moyenne de ses foulées, lorsque ledit individu n'est pas stimulé, lorsque l'intensité de la stimulation est constante, et lorsque l'intensité de stimulation augmente selon l'invention.

**Tableau 1**

| | Durée moyenne (s) | Vitesse moyenne (m/s) | Longueur moyenne des foulées (m) |
|---|---|---|---|
| Sans stimulation | 9,558 | 1,049 | 0,619 |
| Stimulation constante | 9,650 | 1,037 | 0,677 |
| Dispositif selon la variante des figures 1a et 1b | 9,430 | 1,068 | 0,660 |

Comme visible dans le tableau 1, l'invention permet à l'individu de se déplacer plus vite, même si ses foulées sont légèrement moins longues que lorsque l'intensité de stimulation est constante.

Afin de permettre la fixation des capteurs 3 sur le membre inférieur 20 de l'individu 2, le dispositif 1 comprend des éléments d'attache. Un premier élément d'attache permet de fixer un capteur 3 sur la cuisse du membre inférieur 20 de l'individu 2, et un deuxième élément d'attache permet de fixer un autre capteur 3 sur le segment jambier dudit membre inférieur 20. Ainsi, lorsque les capteurs 3 comprennent deux unités de mesures inertielles, une première unité de mesure inertielle est fixée sur la cuisse par le premier élément d'attache, et une deuxième unité de mesure inertielle est fixée sur le segment jambier par le deuxième élément d'attache.

Les éléments d'attache peuvent être formés d'une bande venant s'enrouler autour de la partie désirée du membre inférieur 20 de l'individu 2.

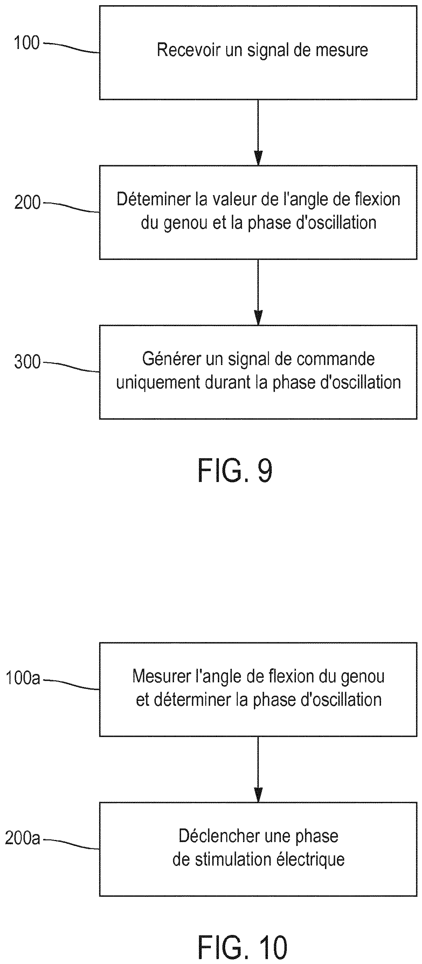

Comme représenté sur la figure 9, le procédé de traitement d'un signal pour l'unité de traitement 50 comprend les étapes suivantes :
- étape 100 : recevoir un signal de mesures de capteurs 3 permettant la mesure d'un angle de flexion du genou 22 du membre inférieur 20 de l'individu 2 ;
- étape 200 : déterminer la valeur de l'angle de flexion du genou 22 à partir du signal de mesure reçu à l'étape 100 précédente et déterminer une phase d'oscillation d'un cycle de marche de l'individu à partir de la valeur de l'angle de flexion du genou ;
- étape 300 : générer un signal de commande uniquement dans la phase d'oscillation détectée lors de l'étape 200 précédente.

Le signal de commande émis par l'unité de traitement permet que les électrodes 4 génèrent une stimulation électrique qui est fonction de l'angle de flexion du genou, et l'intensité de la stimulation électrique augmentant avec la diminution de l'angle de flexion du genou lors de la ré-extension du genou pendant la phase d'oscillation.

Comme représenté sur la figure 10, le procédé de stimulation pour l'activation d'au moins un muscle intervenant dans le relevé du pied lors de la marche d'un individu utilisant le dispositif selon l'invention comprend les étapes suivantes :
- étape 100a : mesurer un angle de flexion du genou 22 de l'individu 2 et déterminer la phase d'oscillation du cycle de marche du membre inférieur 20 de l'individu 2 à partir de la valeur de l'angle de flexion du genou 22 mesurée ;
- étape 200a : déclencher une phase de stimulation électrique du ou des muscles uniquement durant la phase d'oscillation détectée à l'étape 100a précédente, entre une première valeur seuil et une deuxième valeur seuil de l'angle de flexion du genou, l'intensité de la stimulation électrique étant fonction de l'angle de flexion du genou 22, et augmentant avec la diminution de l'angle de flexion du genou lors de la ré-extension du genou 22 pendant la phase d'oscillation.

Selon une caractéristique supplémentaire du procédé de stimulation, la stimulation est déclenchée de façon périodique pour n cycles de marche de l'individu parmi N cycles de marche considérés, n entier naturel supérieur ou égal à 2 et inférieur à strictement à N, N nombre de cycles de marche.

Par exemple, les moyens de commande 53 peuvent déclencher une stimulation un cycle de marche sur deux, pour que l'utilisateur puisse essayer de lui-même de lever le pied, avant d'être à nouveau aidé par la stimulation commandée par l'unité de traitement 5 génératrice de profils de stimulation. Par la suite, l'unité de traitement peut diminuer la fréquence des stimulations, et par exemple déclencher une phase de stimulation un cycle de marche sur trois, puis un cycle de marche sur quatre, et ainsi de suite.

## Revendications

1. Dispositif (1) de stimulation pour l'activation d'au moins un muscle de relevé du pied (21) pour la marche d'un individu (2), comprenant :
- des capteurs (3) configurés pour être mis en place sur un membre inférieur (20) de l'individu (2) et dont des signaux de mesure permettent de calculer un angle de flexion du genou (22) correspondant audit membre inférieur (20) ;
- des électrodes (4) configurées pour être mises en place sur le ou les muscles à activer et propres à stimuler électriquement ledit ou lesdits muscles ;
- une unité de traitement (5) apte à recevoir les signaux de mesure des capteurs (3), l'unité de traitement présentant des moyens de calcul (52) pour calculer la valeur de l'angle de flexion du genou (22) à partir des signaux de mesure , et des moyens de commande (53) des électrodes (4) reliés aux moyens de calcul (52) et aux électrodes (4) ;
où :
• les moyens de calcul (52) sont configurés pour déterminer la phase d'oscillation dans un cycle de marche de l'individu (2) ;
• les moyens de commande sont configurés pour :
- activer les électrodes (4) uniquement dans la phase d'oscillation d'un cycle de marche de l'individu (2) par génération par les électrodes (4) d'une stimulation électrique ayant une intensité fonction de l'angle de flexion du genou,
**caractérisé en ce que** les moyens de commande sont configurés pour :
- activer les électrodes de sorte à générer par les électrodes une stimulation électrique avec une intensité qui augmente avec la diminution de l'angle de flexion du genou (22), à la ré-extension du genou de la phase d'oscillation.

2. Dispositif selon la revendication 1, dans lequel l'unité de traitement (5) est configurée pour déterminer dans la phase d'oscillation une sous-phase de début d'oscillation et une sous-phase de fin d'oscillation, et pour activer les électrodes (4) de telle sorte à générer par les électrodes (4) une stimulation électrique avec une intensité qui augmente entre une première valeur seuil et une deuxième valeur seuil de l'angle du flexion du genou (22), la première valeur seuil étant dans la sous-phase de début d'oscillation, et la deuxième valeur seuil étant dans la sous-phase de fin d'oscillation.

3. Dispositif selon la revendication 1, dans lequel l'unité de traitement (5) est configurée pour activer les électrodes (4) de telle sorte à générer par les électrodes (4) une stimulation électrique avec une intensité qui augmente entre une première valeur seuil et une deuxième valeur seuil, la première valeur seuil étant l'angle de flexion du genou (22) de l'individu (2) au début de la ré-extension du genou (22), et la deuxième valeur seuil étant l'angle de flexion du genou (22) de l'individu (2) à la fin de la ré-extension du genou (22) dans une sous-phase de fin d'oscillation de la phase d'oscillation.

4. Dispositif selon la revendication 3, dans lequel l'unité de traitement (5) est configurée pour activer les électrodes (4) de telle sorte à générer par les électrodes (4) une stimulation électrique ayant une impulsion d'intensité entre une sous-phase de début d'oscillation et le début de l'extension du genou (22).

5. Dispositif selon l'une quelconque des revendications 1 à 4, dans lequel l'unité de traitement (5) est configurée pour commander les électrodes (4) pour générer par lesdites électrodes (4) des impulsions électriques dans la phase d'oscillation, les impulsions électriques étant définies par une amplitude (A) correspondant à l'intensité du courant appliqué aux électrodes (4), l'augmentation de l'intensité de la stimulation dans la phase de stimulation par lesdites électrodes (4) étant une augmentation de l'amplitude (A).

6. Dispositif selon l'une des revendications 1 à 5, dans lequel l'unité de traitement (5) est configurée pour commander les électrodes (4) de façon à ce que l'intensité de la stimulation augmente de façon linéaire avec la diminution de l'angle de flexion à la ré-extension du genou (22).

7. Dispositif selon l'une des revendications 1 à 6, dans lequel l'unité de traitement (5) est configurée pour commander les électrodes (4) de façon à ce que l'intensité de la stimulation augmente de façon linéaire avec l'augmentation de l'angle de flexion à l'augmentation de l'angle de flexion du genou (22) de la phase d'oscillation.

8. Unité de traitement (5) pour un dispositif de stimulation pour l'activation d'au moins un muscle de relevé du pied (21) pour la marche d'un individu (2), configurée pour recevoir des signaux de mesure de capteurs (3), et présentant :
- des moyens de calcul (52) pour déterminer la valeur de l'angle de flexion du genou (22) du membre inférieur (20) de l'individu (2) à partir des signaux de mesure et pour déterminer, la phase d'oscillation dans un cycle de marche de l'individu (2) ;
- des moyens de commande (53) reliés aux moyens de calcul (52) pour commander des électrodes (4) en fonction de la valeur de l'angle de flexion du genou (22) déterminée ;
ou les moyens de commande (53) sont configurés pour générer un signal de commande pour une phase de stimulation par lesdites électrodes (4) uniquement dans la phase d'oscillation du cycle de marche de l'individu (2) :
- de sorte à générer par les électrodes (4) une stimulation électrique dont l'intensité est fonction de l'angle de flexion du genou,
- de sorte à générer par les électrodes (4) une stimulation électrique, **caractérisé en ce que** l'intensité de la stimulation électrique augmente avec la diminution de l'angle de flexion du genou (22), à la ré-extension du genou (22) de la phase d'oscillation.

9. Produit programme d'ordinateur comprenant des instructions de code de programme pour l'exécution des étapes d'un procédé de traitement pour une unité de traitement (5) selon la revendication 8, comprenant les étapes suivantes :
- (100) recevoir des signaux de mesures de capteurs (3) permettant la mesure d'un angle de flexion du genou (22) d'un membre inférieur (20) d'un individu (2) ;
- (200) déterminer la valeur de l'angle de flexion du genou (22) à partir des signaux de mesure et déterminer la phase d'oscillation dans un cycle de marche de l'individu (2) ;
- (300) générer un signal de commande uniquement dans la phase d'oscillation détectée dans l'étape précédente, de sorte à :
- générer par les électrodes (4) une stimulation électrique qui est fonction de l'angle de flexion du genou (22) et dont l'intensité augmente avec la diminution de l'angle de flexion du genou (22), à la ré-extension du genou (22) de la phase d'oscillation ;
lorsque ledit programme est exécuté sur un ordinateur.

10. Support utilisable dans un ordinateur sur lequel est enregistré le produit programme d'ordinateur selon la revendication 9.

## Patentansprüche

1. Stimulationsvorrichtung (1) zur Aktivierung mindestens eines Muskels zum Heben des Fußes (21) für das Gehen eines Individuums (2), umfassend:
- Sensoren (3), die ausgelegt sind, um auf einer unteren Gliedmaße (20) des Individuums (2) platziert zu sein und deren Messsignale erlauben, einen Beugewinkel des Knies (22) entsprechend der unteren Gliedmaße (20) zu berechnen;
- Elektroden (4), die zum Platzieren auf dem oder den zu aktivierenden Muskeln ausgelegt sind und geeignet, den oder die Muskeln elektrisch zu stimulieren;
- eine Verarbeitungseinheit (5), die imstande ist, die Messsignale der Sensoren (3) zu empfangen, wobei die Verarbeitungseinheit Rechenmittel (52) aufweist, um den Wert des Beugewinkels des Knies (22) auf der Basis der Messsignale zu berechnen, und Steuermittel (53) der Elektroden (4), die mit den Rechenmitteln (52) und den Elektroden (4) verbunden sind;
wobei
• die Rechenmittel (52) ausgelegt sind, um die Schwingungsphase in einem Gangzyklus des Individuums (2) zu bestimmen;
• die Steuermittel ausgelegt sind, um:
- die Elektroden (4) nur in der Schwingungsphase eines Gangzyklus des Individuums (2) durch Erzeugen einer elektrischen Stimulation, deren Intensität vom Beugewinkel des Knies abhängt, durch die Elektroden (4) zu aktivieren,
**dadurch gekennzeichnet, dass** die Steuermittel ausgelegt sind, um:
- die Elektroden derart zu aktivieren, dass von den Elektroden eine elektrische Stimulation mit einer Stärke ausgelöst wird, die mit der Verringerung des Beugewinkels des Knies (22) bei der erneuten Streckung des Knies während der Schwingungsphase zunimmt.

2. Vorrichtung nach Anspruch 1, wobei die Verarbeitungseinheit (5) ausgelegt ist, um in der Schwingungsphase eine Unterphase des Schwingungsbeginns und eine Unterphase des Schwingungsendes zu bestimmen, und um die Elektroden (4) derart zu aktivieren, dass von den Elektroden (4) eine elektrische Stimulation mit einer Stärke erzeugt wird, die zwischen einem ersten Grenzwert und einem zweiten Grenzwert des Beugewinkels des Knies (22) zunimmt, wobei der erste Grenzwert in der Unterphase des Schwingungsbeginns liegt und der zweite Grenzwert in der Unterphase des Schwingungsendes liegt.

3. Vorrichtung nach Anspruch 1, wobei die Verarbeitungseinheit (5) ausgelegt ist, um die Elektroden (4) derart zu aktivieren, dass von den Elektroden (4) eine elektrische Stimulation mit einer Stärke erzeugt wird, die zwischen einem ersten Grenzwert und einem zweiten Grenzwert zunimmt, wobei der erste Grenzwert der Beugewinkel des Knies (22) des Individuums (2) zu Beginn der erneuten Streckung des Knies (22) ist und der zweiten Grenzwert der Beugewinkel des Knies (22) des Individuums (2) am Ende der erneuten Streckung des Knies (22) in einer Unterphase des Schwingungsendes des Schwingungsphase ist.

4. Vorrichtung nach Anspruch 3, wobei die Verarbeitungseinheit (5) ausgelegt ist, um die Elektroden (4) derart zu aktivieren, dass von den Elektroden (4) eine elektrische Stimulation mit einem Impuls einer Stärke zwischen einer Unterphase des Schwingungsbeginns und dem Beginn des Streckens des Knies (22) erzeugt wird.

5. Vorrichtung nach einem der Ansprüche 1 bis 4, wobei die Verarbeitungseinheit (5) ausgelegt ist, um die Elektroden (4) zu steuern, um durch die Elektroden (4) elektrische Impulse in der Schwingungsphase zu erzeugen, wobei die elektrischen Impulse durch eine Amplitude (A) definiert sind, die der Stärke des Stroms entspricht, der an die Elektroden (4) angelegt wird, wobei die Erhöhung der Stärke der Stimulation in der Stimulationsphase durch die Elektroden (4) eine Erhöhung der Amplitude (A) ist.

6. Vorrichtung nach einem der Ansprüche 1 bis 5, wobei die Verarbeitungseinheit (5) ausgelegt ist, um die Elektroden (4) derart zu steuern, dass die Stärke der Stimulation mit der Verringerung des Beugewinkels beim erneuten Strecken des Knies (22) linear zunimmt.

7. Vorrichtung nach einem der Ansprüche 1 bis 6, wobei die Verarbeitungseinheit (5) ausgelegt ist, um die Elektroden (4) derart zu steuern, dass die Stärke der Stimulation mit der Vergrößerung des Beugewinkels bei der Vergrößerung des Beugewinkels des Knies (22) der Schwingungsphase linear zunimmt.

8. Verarbeitungseinheit (5) für eine Stimulationsvorrichtung zur Aktivierung mindestens eines Muskels zum Heben des Fußes (21) für das Gehen eines Individuums (2), die ausgelegt ist, um Messsignale von Sensoren (3) zu empfangen, und aufweist:
- Rechenmittel (52), um den Wert des Beugewinkels des Knies (22) der unteren Gliedmaße (20) des Individuums (2) auf der Basis der Messsignale zu bestimmen und um die Schwingungsphase in einem Gangzyklus des Individuums (2) zu bestimmen;
- Steuermittel (53), die mit den Rechenmitteln (52) verbunden sind, um Elektroden (4) in Abhängigkeit von dem bestimmten Wert des Beugewinkels des Knies (22) zu steuern; wobei die Steuermittel (53) ausgelegt sind, um ein Steuersignal für eine Stimulationsphase durch die Elektroden (4) nur in der Schwingungsphase des Gangzyklus des Individuums (2) zu erzeugen:
- derart, dass von den Elektroden (4) eine elektrische Stimulation erzeugt wird, deren Stärke vom Beugewinkel des Knies abhängt,
- derart, dass von den Elektroden (4) eine elektrische Stimulation erzeugt wird, **dadurch gekennzeichnet, dass** die Stärke der elektrischen Stimulation mit der Verringerung des Beugewinkels des Knies (22) bei der erneuten Streckung des Knies (22) während der Schwingungsphase zunimmt.

9. Rechnerprogrammprodukt, umfassend Programmcodebefehle zum Ausführen der Schritte eines Verarbeitungsverfahrens für eine Verarbeitungseinheit (5) nach Anspruch 8, umfassend die folgenden Schritte:
- (100) Empfangen der Messsignale von Sensoren (3), die das Messen eines Beugewinkels des Knies (22) einer unteren Gliedmaße (20) eines Individuums (2) erlauben;
- (200) Bestimmen des Wertes des Beugewinkels des Knies (22) auf der Basis der Messsignale und Bestimmen der Schwingungsphase in einem Gangzyklus des Individuums (2);
- (300) Erzeugen eines Steuersignals nur in der im vorhergehenden Schritt ermittelten Schwingungsphase derart, dass:
- von den Elektroden (4) eine elektrische Stimulation erzeugt wird, die vom Beugewinkel des Knies (22) abhängt und deren Stärke mit der Verringerung des Beugewinkel des Knies (22) bei der erneuten Streckung des Knies (22) während der Schwingungsphase zunimmt; wenn das Programm auf einem Rechner ausgeführt wird.

10. Träger, der in einem Rechner verwendbar ist, auf dem das Rechnerprogrammprodukt nach Anspruch 9 gespeichert ist.

## Claims

1. A stimulation device (1) for activating at least one muscle involved in raising the foot (21) while an individual (2) is walking, comprising:
- sensors (3) configured to be placed on a lower limb (20) of the individual (2) and of which measurement signals allow calculating an angle of flexion of the knee (22) corresponding to said lower limb (20);
- electrodes (4) configured to be placed on the muscle(s) to be activated and suitable for electrically stimulating said muscle(s);
- a processing unit (5) able to receive the measurement signals from the sensors (3), the processing unit having calculation means (52) for calculating the value of the angle of flexion of the knee (22) from the measurement signals, and electrode (4) control means (53) connected to the calculation means (52) and to the electrodes (4);
where
• the calculation means (52) are configured to determine the swing phase in a walking cycle of the individual (2);
• the control means are configured to:
- activate the electrodes (4) only during the swing phase of a walking cycle of the individual (2) so that the electrodes (4) generate electrical stimulation having an intensity as a function of the angle of flexion of the knee,
**characterized in that** the control means are configured to:
- activate the electrodes so that the electrodes generate electrical stimulation with an intensity that increases with the decrease of the angle of flexion of the knee (22), upon re-extension of the knee during the swing phase.

2. The device according to claim 1, wherein the processing unit (5) is configured to determine, in the swing phase, an initial-swing sub-phase and a terminal-swing sub-phase, and to activate the electrodes (4) so as that the electrodes (4) generate electrical stimulation with an intensity that increases between a first threshold value and a second threshold value of the angle of flexion of the knee (22), the first threshold value being in the initial-swing sub-phase, and the second threshold value being reached in the terminal swing sub-phase.

3. The device according to claim 1, wherein the processing unit (5) is configured to activate the electrodes (4) such that the electrodes (4) generate electrical stimulation with an intensity that increases between a first threshold value and a second threshold value, the first threshold value being the angle of flexion of the knee (22) of the individual (2) at the beginning of the re-extension of the knee (22), and the second threshold value being the angle of flexion of the knee (22) of the individual (2) at the end of the re-extension of the knee (22) occurring in a terminal-swing sub-phase of the swing phase.

4. The device according to claim 3, wherein the processing unit (5) is configured to activate the electrodes (4) such that the electrodes (4) generate electrical stimulation having an intensity pulse between an initial-swing sub-phase and the beginning of the extension of the knee (22).

5. The device according to any one of claims 1 to 4, wherein the processing unit (5) is configured to control the electrodes (4) such that said electrodes (4) generate electrical pulses during the swing phase, the electrical pulses being defined by an amplitude (A) corresponding to the intensity of the current applied to the electrodes (4), the increase in the intensity of the stimulation during the stimulation phase by said electrodes (4) being carried out by increasing the amplitude (A).

6. The device according to any of claims 1 to 5, wherein the processing unit (5) is configured to control the electrodes (4) so that the intensity of the stimulation increases linearly with the decrease of the angle of flexion upon re-extension of the knee (22).

7. The device according to any of claims 1 to 6, wherein the processing unit (5) is configured to control the electrodes (4) so that the intensity of the stimulation increases linearly with the increase of the angle of flexion during the increase of the angle of flexion of the knee (22) of the swing phase.

8. A processing unit (5) for a stimulation device for activating at least one muscle involved in raising the foot (21) while an individual (2) is walking, configured to receive measurement signals from the sensors (3), and having:
- calculation means (52) for determining the value of the angle of flexion of the knee (22) of the lower limb (20) of the individual (2) from the measurement signals and for determining the swing phase in a walking cycle of the individual (2);
- control means (53) connected to the calculation means (52) for controlling electrodes (4) as a function of the value of the determined angle of flexion of the knee (22);
where the control means (53) is configured to generate a control signal for a stimulation phase by said electrodes (4) only during the swing phase of the walking cycle of the individual (2):
- so that the electrodes (4) generate electrical stimulation the intensity of which is a function of the angle of flexion of the knee,
- so that the electrodes (4) generate electrical stimulation, **characterized in that** the intensity of the electrical stimulation increases with the decrease of the angle of flexion of the knee (22),upon re-extension of the knee (22) during the swing phase.

9. A computer program product comprising program code instructions for performing the steps of a processing method for a processing unit (5) according to claim 8, comprising the following steps:
- (100) receiving measurement signals from sensors (3) allowing to measure an angle of flexion of the knee (22) of a lower limb (20) of an individual (2);
- (200) determining the value of the angle of flexion of the knee (22) from the measurement signals and determining the swing phase in a walking cycle of the individual (2);
- (300) generating a control signal only during the swing phase detected in the previous step, so that:
- the electrodes (4) generate electrical stimulation which is a function of the angle of flexion of the knee (22) and the intensity of which increases with the decrease of the angle of flexion of the knee (22), upon re-extension of the knee (22) during the swing phase;
when said program is run on a computer.

10. A support usable in a computer on which is recorded the computer program product according to claim 9.
